# EUROPEAN PATENT APPLICATION

(11) **EP 2 213 351 A1**
(43) Date of publication of application: **04.08.2010**
(21) Application number: 09151940.5
(22) Date of filing: 03.02.2009
(51) Int. Cl.: B01D 1/00, B01D 3/00, C07B 63/00, C07C 67/54

(54) **A distillation system and a method**

(71) Applicant: Lindquist, Carl-Johan, 703 58 Örebro (SE)
(72) Inventor: Lindquist, Carl-Johan, 703 58 Örebro (SE)
(74) Representative: Löfgren, Jonas

(57) **Abstract**

A distillation system (1) comprising a distillation vessel (2) with a suction outlet (5) for withdrawing evaporated alcohol from the distillation vessel, a heating device (6) configured to heat a liquid mixture, a vacuum pump (7) configured to create a negative pressure in the distillation vessel, the vacuum pump being connected to the suction outlet of the distillation vessel via a suction channel (8), wherein the distillation system comprises a heat pump comprising an evaporator (9), and a condenser (10), the evaporator being situated in the suction channel and configured to transfer heat from the evaporated alcohol to a heat pump medium and the condenser being situated in the distillation vessel or in an inlet channel (11) of the distillation vessel, the condenser being configured to transfer heat from the heat pump medium to the liquid mixture.

## Description

### FIELD OF THE INVENTION AND BACKGROUND ART

The present invention relates to a distillation system according to the preamble of claim 1 and a method for separation according to the preamble of claim 8.

The invention is applicable to separation of short-chained aliphatic alcohols from fatty acid alkyl esters and/or glycerol in a liquid mixture. The term "short-chained aliphatic alcohol" here refers to an aliphatic alcohol having a molecular formula with just one OH group and less than five carbon atoms, such as for instance methanol, ethanol, propanol or iso-propanol.

In production of fatty acid alkyl esters, i.e. biodiesel, from vegetable oils, animal fast or cooking oil waste, the oils are usually re-esterified by substituting the alcohol (commonly glycerol) in the ester by another alcohol (commonly methanol). There exist numerous such re-esterification processes and most of the processes generally produce a liquid mixture comprising fatty acid alkyl esters and glycerol.

The alcohol in a liquid mixture such as the one mentioned above needs to be separated from the other liquids in the mixture in order to obtain pure biodiesel and this separation is usually performed by distillation. A problem associated with such distillations is that the risk of encountering saponification of the fatty acid alkyl esters increases if the liquid mixture is subjected to high temperatures, and to avoid this risk the distillation can be performed at a relatively low temperature. To do so the evaporation temperature of alcohol has to be lowered and that can be achieved by performing the distillation at reduced pressure. A further reason to decrease the distillation temperature is that the energy consumption decreases when decreasing said distillation temperature.

US 2008/0115407 A1 describes a system and method for producing biodiesel, wherein the distillation of a liquid mixture such as the one described above to separate methanol and/or water from said liquid mixture is performed in an evaporator under reduced pressure. After distillation the remaining liquid is discharged through lines comprising heat exchangers so as to transfer heat from the liquid back to the liquid mixture to be distilled in the evaporator and thereby some of the energy used to heat the liquid mixture in the evaporator can be recycled.

### SUMMARY OF THE INVENTION

The object of the present invention is to provide a new and energy efficient distillation system and method for separation of alcohol from fatty acid alkyl esters and/or glycerol in a liquid mixture.

This object is according to the invention achieved by means of a distillation system having the features defined in claim 1 and a method having the features defined in claim 8.

The evaporated alcohol contains energy in the form of heat and by having the distillation system provided with a heat pump, which heat pump is configured to transfer heat from the evaporated alcohol to the liquid mixture, energy can be recycled and thereby costs can be saved. The evaporator also ensures that the condensation of the evaporated alcohol is efficient. The condensated alcohol can thereafter be re-used in production of biodiesel from vegetable oils, animal fats or cooking oil waste.

According to an embodiment of the invention the distillation system comprises a bubbling device at least partly situated in the distillation vessel and configured to create bubbles or cavities in the liquid mixture received in the distillation vessel.

In this description and the subsequent claims the term "bubbling device" refers to a device which can create bubbles or cavities in a liquid. The device can for instance be a bubbler configured to lead a gas through the liquid, a propeller which rotates in the liquid at such a speed that bubbles/cavities are produced in the liquid or a vibrating device which vibrates in the liquid at frequencies high enough to produce bubbles/cavities in the liquid.

By producing bubbles or cavities in the liquid mixture the active surface of the liquid mixture towards vacuum is enlarged, and thereby the alcohol in the liquid mixture is more easily evaporated. The alcohol is also transported to the surface of the liquid mixture by the bubbles, which further facilitates the evaporation of alcohol. Another advantage obtained by producing bubbles or cavities in the liquid mixture in the distillation vessel is that boiling stones, usually added during distillation to prevent flashing, can be avoided. This is a major advantage since it can be difficult to remove the boiling stones from the distillation vessel, for instance when they need to be replaced.

According to another embodiment of the invention the bubbling device is an ultra sonic generator. An ultra sonic generator generates ultra sonic waves in the liquid to thereby produce bubbles or cavities. The energy consumption of the ultra sonic generator is relatively low when considering the decrease in evaporation temperature of the alcohol from the liquid mixture; hence the total energy consumption of the distillation system is reduced.

According to another embodiment of the invention the distillation system further comprises:
■ a heat exchanging circuit comprising a first heat exchanger, a second heat exchanger and a heat exchanging medium circulated in the heat exchanging circuit by a pump, the first and the second heat exchanger being connected to each other in the heat exchanging circuit, and
■ a collecting vessel, here denominated first collecting vessel, situated below the distillation vessel and connected to the discharge outlet of the distillation vessel via a first discharge channel, and configured to receive liquid from the distillation vessel, the first heat exchanger being situated in the first collecting vessel and configured to transfer heat from the liquid in the first collecting vessel to the heat exchanging medium, and the second heat exchanger being situated in the inlet channel or in a pre-heat chamber connected to the inlet channel, the second heat exchanger being configured to transfer heat from the heat exchanging medium to the liquid mixture in the inlet channel or pre-heat chamber.

By arranging the first collecting vessel below the distillation vessel there is no need for a pump for transporting the liquid from the distillation vessel to the first collecting vessel. Energy is thereby saved in the system. Energy is also saved by having the distillation system provided with a heat exchanging circuit, since energy, in the form of heat, present in the liquid can be recycled to some extent by using the heat for pre-heating the liquid mixture prior to introducing it into the distillation vessel.

According to another embodiment of the invention the distillation system is **characterized in:**
■ **that** the distillation system comprises a second collecting vessel situated below the first collecting vessel, the second collecting vessel being connected to the first collecting vessel via a second discharge channel,
■ that the distillation system comprises a first control valve configured to open and close said second discharge channel,
■ that the second collecting vessel is configured to receive liquid from the first collecting vessel when the first control valve is open,
■ that the second collecting vessel is connected to the distillation vessel by an air channel, and
■ that the distillation system comprises a second control valve configured to open and close the air channel, the air channel being configured to lead air from the second collecting vessel to the distillation vessel so as to achieve a negative pressure inside the second collecting vessel when the second control valve is open.

In order to save energy it is important that the distillation is performed under negative pressure and therefore it is important that the negative pressure in the distillation vessel is maintained. The distillation vessel is configured to be discharged directly into the first collecting vessel, and therefore the first collecting vessel has the same negative pressure as the distillation vessel. If discharging the liquid directly to atmospheric pressure from the first collecting vessel, the pressure in the distillation vessel and said first collecting vessel will increase to atmospheric pressure. When discharging the liquid from the first collecting vessel to the second collecting vessel it is therefore important that the negative pressure in the first collecting vessel is maintained; and therefore discharging is performed only when the second collecting vessel has essentially the same negative pressure as the first collecting vessel and the distillation vessel. Negative pressure in the second collecting vessel is achieved by transporting air from the second collecting vessel to the distillation vessel in the air channel. The vacuum pump thus only needs to evacuate an air volume corresponding to the volume in the second collecting vessel, which usually is smaller than the added volumes of the distillation vessel and the first collecting vessel. If the system instead were adapted to ventilate the distillation vessel and the first collecting vessel when discharging a liquid from said distillation vessel, an air volume corresponding to those vessels would need to be evacuated by the vacuum pump before another load of the liquid mixture could be distilled under vacuum in the distillation vessel. By having the distillation system equipped with the discharging system mentioned above it is only necessary to evacuate the second collecting vessel between each load of liquid discharged.

According to another embodiment of the invention the heat exchanging circuit comprises a third heat exchanger, which is connected to the second heat exchanger and situated in the second collecting vessel and configured to transfer heat from the liquid in the second collecting vessel to the heat exchanging medium. By providing the distillation system with a further heat exchanger in the second collecting vessel, heat can be transferred back to the liquid mixture from the liquid passing the second collecting vessel. Thereby the total energy consumption in the distillation system is lowered.

According to another embodiment of the invention the distillation system is **characterized in:**
■ **that** the distillation system comprises a washing tank situated below the second collecting vessel, the washing tank being connected to the second collecting vessel via a third discharge channel,
■ that the distillation system comprises a third control valve configured to open and close said third discharge channel,
■ that the washing tank is configured to receive liquid from the second collecting vessel when the third control valve is open, and
■ that the heat pump comprises a second evaporator situated in the washing tank and configured to transfer heat from the liquid in the washing tank to the heat pump medium.

The liquid transported from the distillation vessel contains contaminants such as salts and other water soluble compounds.

In the washing tank the liquid is washed, e.g by ultra sonic washing or by stirring the liquid in for instance water, and thereafter the water phase is separated from the rest of the liquid for instance by fractionation. The water soluble contaminants are thereby removed. Heat is also transferred from the liquid and/or the water in the washing tank to the liquid mixture in the distillation vessel by using the heat pump, and energy is thereby saved.

Other advantages and advantageous features of the invention will appear from the dependent claims and the subsequent description.

### BRIEF DESCRIPTION OF THE DRAWINGS

With reference to that appended drawings, below follows a specific description of embodiments of the invention cited as examples.

In the drawing:
- Fig 1: shows a first embodiment of a distillation system according to the invention in a schematic view,
- Fig 2: shows a second embodiment of a distillation system according to the invention in a schematic view,
- Fig 3: shows a third embodiment of a distillation system according to the invention in a schematic view, and
- Fig 4: shows a fourth embodiment of a distillation system ac- cording to the invention in a schematic view.

### DETAILED DESCRIPTION OF PREFERRED EMBODIMENTS OF THE INVENTION

Explained herein are preferred embodiments of the invention, describing the distillation system and the method for separation of the invention. The invention may, however, be embodied in many different forms and should not be construed as being limited to the exemplary embodiments set forth herein; rather, these embodiments are provided so that this disclosure will be thorough and complete, and will fully convey the concept of the invention to those skilled in the art.

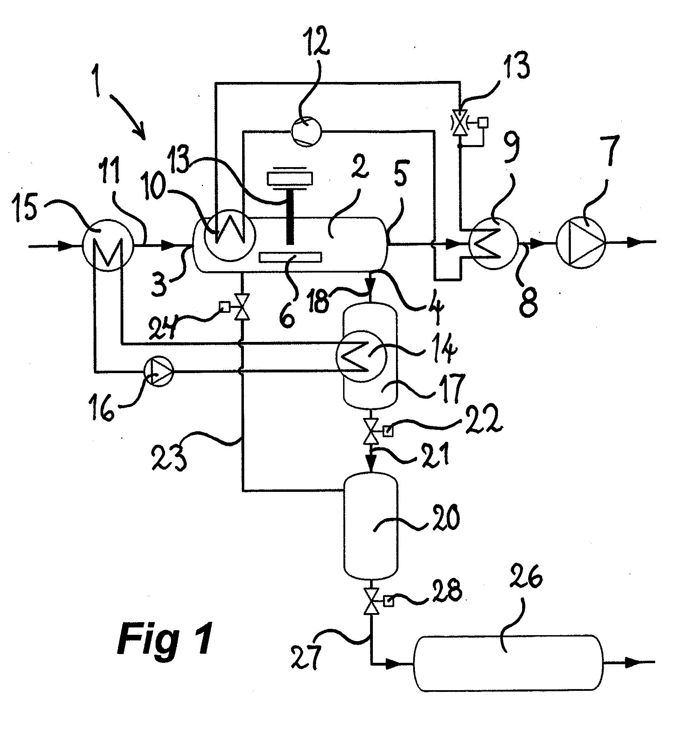

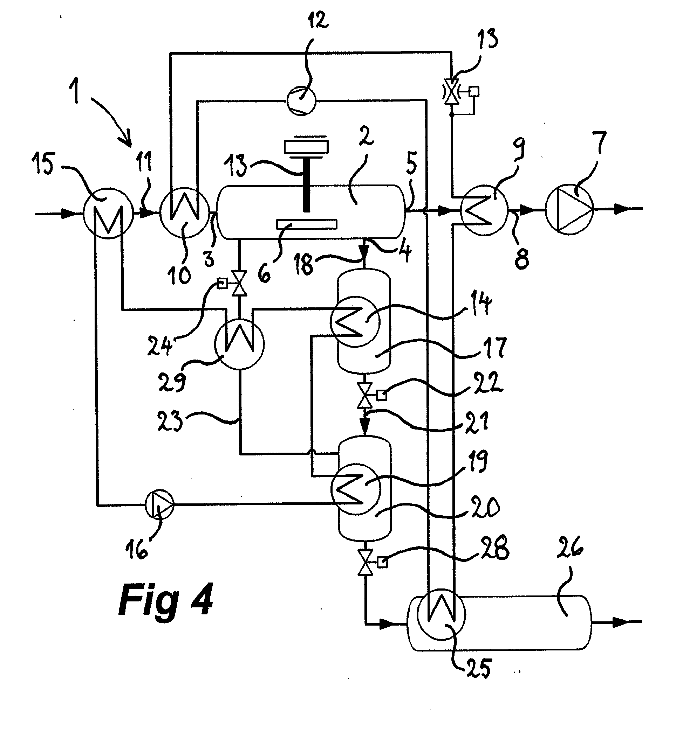

The inventive distillation system 1 illustrated in Figs 1 - 4 comprises a distillation vessel 2 with an inlet 3 for introducing the liquid mixture to the distillation vessel 2, a discharge outlet 4 for discharging liquid from the distillation vessel 2, a suction outlet 5 for withdrawing evaporated alcohol from the distillation vessel 2, a heating device 6, e.g. an electrical heating element, configured to heat the liquid mixture received in the distillation vessel 2, a vacuum pump 7 configured to create a negative pressure in the distillation vessel 2, the vacuum pump 7 being connected to the suction outlet 5 of the distillation vessel 2 via a suction channel 8. The distillation system further comprises a heat pump comprising an evaporator, here denominated first evaporator 9, and a condenser 10. The first evaporator 9 is situated in the suction channel 8 and configured to transfer heat from the evaporated alcohol to a heat pump medium. The condenser 10 is situated in the distillation vessel 2 as illustrated in Figs 1-3, or situated in an inlet channel 11 leading to the inlet 3 of the distillation vessel 2 as illustrated in Fig 4. The condenser 10 can also be an integrated part of the heating device 6. The condenser 10 is configured to transfer heat from the heat pump medium to the liquid mixture. By heat exchange with evaporated alcohol in the suction channel 8, the heat pump medium absorbs heat energy via the first evaporator 9. Work is added to the heat pump via a compressor 12, whereby the pressure and the temperature of the heat pump medium of the heat pump is increased. In the condenser 10, heat energy is then emitted to the liquid mixture in the distillation vessel 2 by heat exchange between the heat pump medium and the liquid mixture and the heat pump medium is then returned to the first evaporator 9 via an expansion valve 13, preferably an electromechanical expansion valve, while being subjected to pressure and temperature decrease. The evaporated alcohol condenses on the first evaporator 9. The heat energy from the evaporated alcohol is transferred to the heat pump medium in the first evaporator 9. The heat pump medium is transported in the circuit of the heat pump to the condenser 10 in the distillation vessel 2, wherein the heat energy in the heat pump medium is transferred to the liquid mixture in the distillation vessel 2 by said condenser 10.

The inventive distillation system 1 as illustrated in Figs 1 - 4 also comprises a bubbling device 13, preferably an ultra sonic generator, at least partly situated in the distillation vessel 2 and configured to create bubbles or cavities in a liquid mixture received in the distillation vessel 2. A collecting vessel, here denominated first collecting vessel 17, is situated below the distillation vessel 2 and is connected to the discharge outlet 4 of the distillation vessel 2 via a first discharge channel 18. The first collecting vessel 17 is configured to receive the liquid from the distillation vessel 2. A second collecting vessel 20 is situated below the first collecting vessel 17. The second collecting vessel 20 is connected to the first collecting vessel 17 via a second discharge channel 21. The distillation system 1 also comprises a first control valve 22 configured to open and close said second discharge channel 21. The second collecting 20 vessel is configured to receive liquid from the first collecting vessel 17 when the first control valve 22 is open and when the pressure in the second collecting vessel 20 is essentially the same as the pressure in the first collecting vessel 17 and the distillation vessel 2. A washing tank 26 is situated below the second collecting vessel 20 and is connected to the second collecting vessel 20 via a third discharge channel 27. The distillation system 1 also comprises a third control valve 28 configured to open and close said third discharge channel 27. The washing tank 26 is configured to receive liquid from the second collecting vessel 20 when the third control valve 28 is open. During operation of the distillation system 1 there is atmospheric pressure in the washing tank 26, so when the washing tank 26 receives the liquid from the second collecting vessel 20, said second collecting vessel 20 is ventilated to atmospheric pressure. The second collecting 20 vessel is also connected to the distillation vessel 2 by an air channel 23, and the distillation system 1 comprises a second control valve 24 configured to open and close the air channel 23. The air channel 23 is configured to lead air from the second collecting vessel 20 to the distillation vessel 2 so as to recreate the negative pressure inside the second collecting vessel 20 when the second control valve 24 is open and after the liquid has been discharged from the second collecting vessel 20 to the washing tank 26. The first, second and third control valves 22, 24, 28 can all be controlled automatically by an electronic control unit (not shown) during operation of the distillation system, i.e.:
■ the first control valve 22 is opened when the pressure in the second collecting vessel 20 is essentially the same as the pressure in the first collecting vessel 17 and the liquid in the first collecting vessel reaches a certain level,
■ the third control valve 28 is opened and the first control valve 22 is closed when the liquid in the second collecting vessel 20 reaches a certain level,
■ and the second control valve 24 is opened when the third control valve 28 is closed and thereafter closed when the pressure in the second collecting vessel 20 is essentially the same as the pressure in the first collecting vessel 17.

In all of the illustrated embodiments the distillation system further comprises a heat exchanging circuit comprising a first heat exchanger 14, a second heat exchanger 15 and a heat exchanging medium circulated in the heat exchanging circuit by a pump 16, the first and the second heat exchanger 14, 15 being connected to each other in the heat exchanging circuit. The first heat exchanger 14 is situated in the first collecting vessel 17 and configured to transfer heat from the liquid in the first collecting vessel 17 to the heat exchanging medium, and the second heat exchanger 15 is situated in the inlet channel 11 or in a pre-heat chamber connected to the inlet channel 11. The second heat exchanger 15 is configured to transfer heat from the heat exchanging medium to the liquid mixture in the inlet channel 11 or pre-heat chamber.

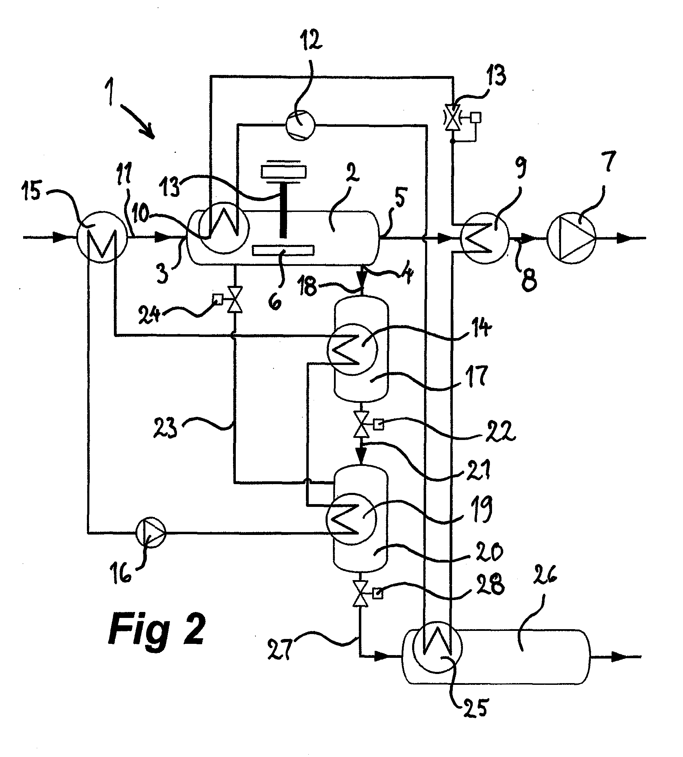

In the embodiments illustrated in Figs 2-4 a third heat exchanger 19 is connected to the second heat exchanger 15 and situated in the second collecting vessel 20 and configured to transfer heat from the liquid in the second collecting vessel 20 to the heat exchanging medium. The heat is then transferred to the liquid mixture in the inlet channel 11 or in a pre-heat chamber connected to the inlet channel 11 via the heat exchanging medium by the second heat exchanger 15. As illustrated in Figs 2-4 the third heat exchanger 19 is connected in series with the first heat exchanger 14 and the second exchanger 15, i.e. the heat exchanging medium is first heated in the third heat exchanger 19 and thereafter more heated in the first heat exchanger 14, whereafter the heat in the heat exchanging medium is transferred to the liquid mixture in the second heat exchanger 15. The embodiment illustrated in Fig 1 can of course also comprise such a third heat exchanger 19.

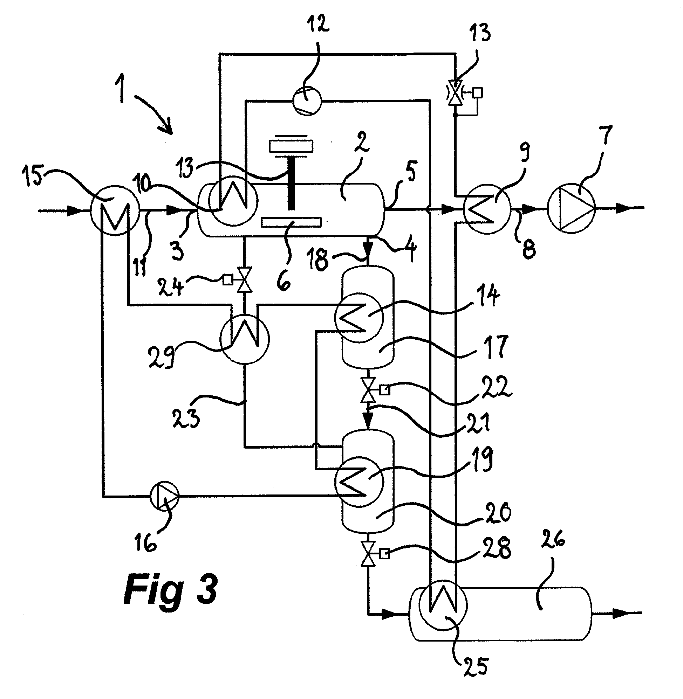

The illustrated embodiments of Figs 3-4 also comprises an optional fourth heat exchanger 29 situated in the air channel 23, connected to the heat exchanging circuit and configured to transfer heat from the heat exchanging medium to air passing through the air channel 23 when the second control valve 24 is open. As illustrated in Figs 3-4 the fourth heat exchanger 29 is connected in series with the first heat exchanger 14, the second exchanger 15 and the third heat exchanger 19, i.e. the heat exchanging medium is first heated in the third heat exchanger 19 and thereafter more heated in the first heat exchanger 14, whereafter heat from the heat exchanging medium is transferred to air passing through the air channel 23 in the fourth heat exchanger 29, and after that heat in the heat exchanging medium is transferred to the liquid mixture in the second heat exchanger 15. The embodiments illustrated in Figs 1-2 can of course also comprise such a fourth heat exchanger 29.

In the embodiments illustrated in Figs 2-4 a second evaporator 25 is situated in the washing tank 26 and configured to transfer heat from a liquid in the washing tank 26 to the heat pump medium. The second evaporator 25 is connected in series with the first evaporator 9, i.e. the heat pump medium is first heated in the first evaporated 9 and then further heated in the second evaporator 25. The embodiment illustrated in Fig 1 can of course also comprise such a second evaporator 25.

The distillation vessel 2 can also be equipped with a barrier (not shown) at its bottom configured to prevent solid contaminant particles in the liquid mixture from reaching the discharge outlet, and the distillation vessel 2 can also be tiltable about a horizontal axis to allow liquid to flow over the barrier and out from the distillation vessel through the discharge outlet.

The invention is of course not in any way limited to the embodiments described above. On the contrary, several possibilities to modifications thereof should be apparent to a person skilled in the art without departing from the basic idea of the invention as defined in the appended claims.

## Claims

1. A distillation system (1) for separation of alcohol from fatty acid alkyl esters and/or glycerol in a liquid mixture, the distillation system comprising:
■ a distillation vessel (2) with an inlet (3) for introducing the liquid mixture to the distillation vessel (2), a discharge outlet (4) for discharging liquid from the distillation vessel (2), a suction outlet (5) for withdrawing evaporated alcohol from the distillation vessel (2),
■ a heating device (6) configured to heat the liquid mixture received in the distillation vessel (2),
■ a vacuum pump (7) configured to create a negative pressure in the distillation vessel (2), the vacuum pump (7) being connected to the suction outlet (5) of the distillation vessel (2) via a suction channel (8),
**characterized in that** the distillation system (1) comprises a heat pump comprising an evaporator, here denominated first evaporator (9), and a condenser (10), the first evaporator (9) being situated in the suction channel (8) and configured to transfer heat from the evaporated alcohol to a heat pump medium and the condenser (10) being situated in the distillation vessel (2) or in an inlet channel (11) leading to the inlet (3) of the distillation vessel (2), the condenser (10) being configured to transfer heat from the heat pump medium to the liquid mixture.

2. A distillation system (1) according to claim 1, **characterized in that** the distillation system (1) comprises a bubbling device (13) at least partly situated in the distillation vessel (2) and configured to create bubbles or cavities in a liquid mixture received in the distillation vessel (2).

3. A distillation system (1) according to claim 2, **characterized in that** the bubbling device (13) is an ultra sonic generator.

4. A distillation system (1) according to any of the preceding claims, **characterized in that** the distillation system (1) further comprises:
■ a heat exchanging circuit comprising a first heat exchanger (14), a second heat exchanger (15) and a heat exchanging medium circulated in the heat exchanging circuit by a pump (16), the first and the second heat exchanger (14, 15) being connected to each other in the heat exchanging circuit, and
■ a collecting vessel, here denominated first collecting vessel (17), situated below the distillation vessel (2) and connected to the discharge outlet (4) of the distillation vessel (2) via a first discharge channel (18), and configured to receive the liquid from the distillation vessel (2), the first heat exchanger (14) being situated in the first collecting vessel (17) and configured to transfer heat from the liquid in the first collecting vessel (17) to the heat exchanging medium, and the second heat exchanger (15) being situated in the inlet channel (11) or in a pre-heat chamber connected to the inlet channel (11), the second heat exchanger (15) being configured to transfer heat from the heat exchanging medium to the liquid mixture in the inlet channel (11) or pre-heat chamber.

5. A distillation system (1) according to claim 4, **characterized in:**
■ **that** the distillation system (1) comprises a second collecting vessel (20) situated below the first collecting vessel (17), the second collecting vessel (20) being connected to the first collecting vessel (17) via a second discharge channel (21),
■ that the distillation system (1) comprises a first control valve (22) configured to open and close said second discharge channel (21),
■ that the second collecting vessel (20) is configured to receive liquid from the first collecting vessel (17) when the first control valve (22) is open,
■ that the second collecting vessel (20) is connected to the distillation vessel (2) by an air channel (23), and
■ that the distillation system (1) comprises a second control valve (24) configured to open and close the air channel (23), the air channel (23) being configured to lead air from the second collecting vessel (20) to the distillation vessel (2) so as to achieve a negative pressure inside the second collecting vessel (20) when the second control valve (24) is open.

6. A distillation system (1) according to claim 5, **characterized in that** the heat exchanging circuit comprises a third heat exchanger (19), which is connected to the second heat exchanger (15) and situated in the second collecting vessel (20) and configured to transfer heat from the liquid in the second collecting vessel (20) to the heat exchanging medium.

7. A distillation system (1) according to claim 5 or 6, **characterized in:**
■ **that** the distillation system (1) comprises a washing tank (26) situated below the second collecting vessel (20), the washing tank (26) being connected to the second collecting vessel (20) via a third discharge channel (27),
■ that the distillation system (1) comprises a third control valve (28) configured to open and close said third discharge channel (27),
■ that the washing tank (26) is configured to receive liquid from the second collecting vessel (20) when the third control valve (28) is open, and
■ that the heat pump comprises a second evaporator (25) situated in the washing tank (26) and configured to transfer heat from the liquid in the washing tank (26) to the heat pump medium.

8. A method for separation of alcohol from fatty acid alkyl esters and/or glycerol in a liquid mixture, the method comprising the steps of:
■ introducing the liquid mixture to a distillation vessel (2) through an inlet (3) in the distillation vessel (2), and
■ distillation of the liquid mixture under negative pressure in the distillation vessel (2) to evaporate alcohol from the liquid mixture and withdrawing the evaporated alcohol from the distillation vessel (2) through a suction outlet (5) and into a suction channel (8),
**characterized in that** the method comprises the step of:
■ transferring heat from the evaporated alcohol to the liquid mixture in the distillation vessel (2), or to the liquid mixture in an inlet channel (11) leading to the inlet (3) of the distillation vessel, by means of a heat pump comprising an evaporator, here denominated first evaporator (9), situated in the suction channel (8) and a condenser (10) situated in the distillation vessel (2) or in the inlet channel (11), wherein the first evaporator (9) transfers heat from the evaporated alcohol to a heat pump medium and the condenser (10) transfers heat from the heat pump medium to the liquid mixture.

9. A method according to claim 8, **characterized in that** bubbles or cavities are created in the liquid mixture in the distillation vessel (2) by means of a bubbling device (13), at least partly situated in the distillation vessel (2).

10. A method according to claim 9, **characterized in that** bubbles or cavities are created in the liquid mixture in the distillation vessel (2) by means of a bubbling device (13) in the form of an ultra sonic generator, at least partly situated in the distillation vessel (2).

11. A method according to any of claims 8-10, **characterized in that** the method comprises the steps of:
■ discharging liquid from the distillation vessel (2) to a collecting vessel, here denominated first collecting vessel (17), situated below the distillation vessel (2) and connected to a discharge outlet (4) of the distillation vessel (2) via a first discharge channel (18), and
■ transferring heat from the liquid in the first collecting vessel (17) to the liquid mixture prior to introducing the liquid mixture to the distillation vessel (2) by means of a heat exchanging circuit which comprises a first heat exchanger (14) situated in the first collecting vessel (17), a second heat exchanger (15) situated in the inlet channel (11) connected to the inlet (3) of the distillation vessel (2) or situated in a pre-heat chamber connected to said inlet channel (11), and a heat exchanging medium circulated in the heat exchanging circuit by a pump (16), the first and the second heat exchanger (14, 15) being connected to each other in the heat exchanging circuit, wherein heat from the liquid in the first collecting vessel (17) is transferred to the heat exchanging medium by the first heat exchanger (14) and heat from the heat exchanging medium is transferred to the liquid mixture by the second heat exchanger (15).

12. A method according to claim 11, **characterized in that** the method comprises the steps of:
■ discharging liquid from the first collecting vessel (17) to a second collecting vessel (20) situated below the first collecting vessel (17) and connected to the first collecting vessel (17) via a second discharge channel (21), and
■ transferring heat from the liquid in the second collecting vessel (20) to the heat exchanging medium by means of a third heat exchanger (19) situated in the second collecting vessel (20) and connected to the second heat exchanger (15) by the heat exchanging circuit.

13. A method according to claim 12, **characterized in that** the method comprises the steps:
■ discharging liquid from the second collecting vessel (20) to a washing tank (26) situated below the second collecting vessel (20) and connected to the second collecting vessel (20) via a third discharge channel (27), and
■ transferring heat from the liquid in the washing tank (26) to the heat pump medium by means of a second evaporator (25) of the heat pump situated in the washing tank (26).
